# EUROPEAN PATENT SPECIFICATION

(11) **EP 3 443 971 B1**
(45) Date of publication and mention of the grant of the patent: **27.08.2025**
(21) Application number: 17781921.6
(22) Date of filing: 13.04.2017
(51) Int. Cl.: A61K 36/076, C07J 9/00, A61P 21/00

(54) **USES OF PORIA COCOS EXTRACT AND TUMULOSIC ACID IN PROTECTING MUSCLES**
VERWENDUNG EINES PORIA-COCOS-EXTRAKTS UND TUMULOSINSÄURE ZUM SCHUTZ DER MUSKELN
UTILISATIONS D'UN EXTRAIT DE PORIA COCOS ET D'ACIDE TUMULOSIQUE DANS LA PROTECTION DES MUSCLES

(30) Priority: 14.04.2016 US 201662322406 P
(43) Date of publication of application: 20.02.2019
(73) Proprietor: Sinphar Tian-li Pharmaceutical Co., Ltd. (Hangzhou), Yuhang Economic Development Zone Hangzhou Zhejiang 311100 (CN)
(72) Inventor: WANG, Chao-jih, Hangzhou Zhejiang 311100 (CN); KUO, Han-peng, Yilan County Taiwan (TW); YEH, Ai-ling, Yilan County Taiwan (TW)
(74) Representative: dompatent von Kreisler Selting Werner - Partnerschaft von Patent- und Rechtsanwälten mbB
(86) International application number: PCT/CN2017/080446
(87) International publication number: WO 2017/177945

(56) References cited:
- CN-A- 101 095 938
- CN-A- 104 825 525
- DATABASE WPI Week 200839, Derwent World Patents Index; AN 2008-G05097, XP002795721
- LI WEIFENG ET AL: "Polysaccharides from Poria cocos (PCP) inhibits ox-LDL-induced vascular smooth muscle cells proliferation and migration by suppressing TLR4/NF-[kappa]B p65 signaling pathway", JOURNAL OF FUNCTIONAL FOODS, ELSEVIER BV, NL, vol. 60, 13 June 2019 (2019-06-13), XP085749136, ISSN: 1756-4646, [retrieved on 20190613], DOI: 10.1016/J.JFF.2019.05.047
- RÍOS JOSÉ-LUIS: "Chemical constituents and pharmacological properties of Poria cocos", PLANTA MEDICA, THIEME VERLAG, DE, vol. 77, no. 7, 1 May 2011 (2011-05-01), pages 681 - 691, XP002666237, ISSN: 0032-0943, [retrieved on 20110223], DOI: 10.1055/S-0030-1270823
- ZHAO, XIAOPING ET AL.: "Evaluation of the mechanism of anti-sports fatigue of traditional Chinese medicine", SCIENCE & TECHNOLOGY INFORMATION, vol. 3, no. 16, 3 June 2011 (2011-06-03), pages 67, XP009512622, ISSN: 1672-3791
- ZHAO, XIAOPING ET AL.: "Evaluation of the mechanism of anti-sports fatigue of traditional Chinese medicine", SCIENCE & TECHNOLOGY INFORMATION, vol. 3, no. 16, 3 June 2011 (2011-06-03), pages 67, XP009512622
- ZHANG, XIANSHU ET AL.: "Research Progress of Triterpenes Extracted from Poria cocos(Schw.) Wolf", JOURNAL OF CHONGQING INDUSTRY & TRADE POLYTECHNIC, 1 January 2011 (2011-01-01), pages 46 - 50, XP055579611

## Description

### FIELD OF THE INVENTION

The present invention relates to the uses of a FU-LING (*Poria cocos*) extract and tumulosic acid (i.e., an ingredient of the FU-LING extract) or a pharmaceutically acceptable salt of tumulosic acid. The invention especially relates to the use of the FU-LING extract, tumulosic acid or a pharmaceutically acceptable salt of tumulosic acid in protecting muscles. This includes protecting muscle cells against injury, promoting the regeneration and repair of muscles and thereby regulating, treating, and/or delaying muscle atrophy, especially muscle atrophy caused by aging, diseases, and/or cachexia.

### BACKGROUND OF THE INVENTION

Muscle tissue is the most abundant tissue in mammals, and is mainly responsible for generating force to cause movement of various parts of the body. Muscle can be divided into three groups: skeletal muscle, cardiac muscle and smooth muscle. Based on the metabolic types and characteristics, skeletal muscle can be divided into slow twitch muscle and fast twitch muscle, wherein the former consists of slow-twitch fiber proteins that can twitch for a longer period, but the generated force is weaker; and the latter consists of fast-twitch fiber proteins that can twitch faster and stronger than the former, but fatigues more easily.

Under normal physiological conditions, there is a dynamic balance between the synthesis and degradation of muscle proteins. However, when an imbalance of muscle protein metabolism occurs (namely, the degradation rate of muscle proteins become greater than the synthesis rate), it causes muscle atrophy (or the loss of muscle) and leads to characteristic changes, such as a decrease in muscle mass, a reduction of muscle fiber cross-sectional area, and a selective reduction of muscle fiber type-related proteins (i.e., slow-twitch fiber proteins and fast-twitch fiber proteins), which can result in symptoms that seriously affect daily work and vital function, including a reduction in muscle strength, movement disorders, fatigue, metabolic disturbances, etc.

**In** general, the injured position of skeletal muscle can be repaired through a muscle regeneration process. However, when the regeneration process is severely hampered, muscle atrophy occurs. Researches have found that the muscle regeneration process is quite complex, first involving the activation of satellite cells followed by the differentiation of the activated satellite cells into mononucleated myoblasts. The mononucleated myoblasts further proliferate and differentiate to fuse into multinucleated myotubes, and finally, mature myotubes differentiate into new myofibers. Different stages of muscle regeneration process are respectively regulated by different myogenic regulatory factors (MRFs). For example, in the process of myoblasts differentiating into myotubes, MyoD and myogenin can regulate the formation of myosin heavy chain (MyHC, a myotube-specific structure protein). The aforementioned regulation is an important step in muscle regeneration process, therefore, MyoD, myogenin and MyHC can be used as markers for myoblast differentiation.

It is known that some physiological conditions or specific diseases may cause muscle cell injury, leading to an imbalance of muscle protein metabolism or apoptosis of muscle cells, and ultimately, cause muscle atrophy. Physiological conditions causing muscle atrophy include, for example, neurodegeneration, long-term bed rest, aging, diseases, cachexia (e.g., cancer cachexia), etc.; and specific diseases causing muscle atrophy include sepsis, acquired immune deficiency syndrome (AIDS), renal failure, Cushing's syndrome (CS), sarcopenia, cancer, chronic obstructive pulmonary disease (COPD), congestive heart failure (CHF), etc.

Aging is the main factor causing sarcopenia. Statistical data indicate that the incidence rate of sarcopenia among people aged 60 to 70 is 13 to 24%, while the incidence rate of sarcopenia among people older than 80 years of age is about 50%. In the United States, the medical cost caused by sarcopenia per year is about 11.8 to 26.2 billion USD. While cachexia relates to other diseases with high incidence rates, for example, about 50% of cancer patients, 20 to 40% of COPD patients, and 50 to 70% of CHF patients will exhibit the symptoms of cachexia (e.g., dystrophy).

In clinical practice, there is still a lack of an effective method for treating or delaying muscle atrophy. Therefore, to try to develop a more effective method for treating or delaying muscle atrophy, the inventors of the present invention selected FU-LING from traditional Chinese herbs, and then investigated the feasibility of using FU-LING in protecting muscle (namely, protecting muscle cells against injury and promoting the regeneration and repair of muscles). Said FU-LING, a traditional Chinese herbal medicine, has therapeutic value of medicinal in many diseases. For example, CN101095938A discloses FU-LING as one of components in a composition for use in the treatment of diabetic muscular atrophy. Li Weifeng et al discloses polysaccharides, which s a main active component in FU-LING, inhibits ox-LDL-induced vascular smooth muscle cell proliferation and migration (Journal of Functional Foods, 60, 2019, 103391). José-Luis Ríos discloses anti-inflammatory and immunostimulant effects of FU-LING (Planta Medica, 77, 2011, 681-691).

The inventors of the present invention discovered that the FU-LING and tumulosic acid contained therein are effective in protecting muscle cells against injury and promoting the regeneration and repair of muscles. Therefore, the FU-LING extract and tumulosic acid can be used for regulating, treating and/or delaying muscle atrophy, especially for regulating, treating and/or delaying muscle atrophy caused by aging, disease and/or cachexia, and thus can be used for providing a pharmaceutical composition, a medicament or a food product for protecting muscles.

### SUMMARY OF THE INVENTION

The present invention provides a FU-LING extract defined in claim 1, use of a FU-LING extract in a food product defined in claim 7, an active ingredient defined in claim 8 and use of an active ingredient in a food product defined in claim 12. Preferred embodiments are reflected in dependent claims.

An objective of the present invention is to provide a FU-LING extract for use in treating, delaying and/or regulating muscle atrophy by protecting muscle cells against injury and/or promoting the regeneration and repair of muscles. Preferably, the FU-LING extract is a polar solvent extract of FU-LING, wherein the polar solvent is selected from the group consisting of water, C1-C4 alcohols, and combinations thereof. More preferably, the FU-LING extract comprises tumulosic acid. More preferably, the FU-LING extract is an extract of FU-LING meat, an extract of FU-LING epidermis and/or an extract of FU-LING fermentation product The medicament is used for protecting muscle cells against injury, promoting the regeneration and repair of muscles, or for treating and/or delaying muscle atrophy caused by at least one of the following: aging, disease, and cachexia.

An objective of the present invention is to provide use of a FU-LING extract in a food product for helping normal muscle contraction, maintaining normal muscle physiology, maintaining normal neuromuscular function, maintaining normal energy metabolism, or enhancing energy level. The food product is a health food, a nutritional supplement food or a special nutrition food.

Another objective of the present invention is to provide a medical use of an active ingredient in treating, delaying and/or regulating muscle atrophy by protecting muscles against injury and/or promoting the regeneration and repair of muscles, wherein the active ingredient is tumulosic acid and/or a pharmaceutically acceptable salt of tumulosic acid. Preferably, the active ingredient is used as a plant extract; more preferably, the active ingredient is used as a FU-LING extract, (e.g., the extract of FU-LING meat, FU-LING epidermis and/or FU-LING fermentation product), especially used as a polar solvent extract of FU-LING, wherein the polar solvent is selected from the group consisting of water, C1-C4 alcohols, and combinations thereof. The medicament is used for protecting muscle cells against injury, promoting the regeneration and repair of muscles, or for treating and/or delaying muscle atrophy caused by at least one of the following: aging, disease, and cachexia.

Still another objective of the present invention is to provide use of an active ingredient in a food product for helping normal muscle contraction, maintaining normal muscle physiology, maintaining normal neuromuscular function, maintaining normal energy metabolism, or enhancing energy level, wherein the active ingredient is tumulosic acid and/or a pharmaceutically acceptable salt of tumulosic acid. The food product is a health food, a nutritional supplement food or a special nutrition food.

Described is a method for protecting muscles, comprising administering to a subject in need an effective amount of the active ingredient or FU-LING extract described above. The method is for protecting muscle cells against injury, promoting the regeneration and repair of muscles, or for regulating, treating, and/or delaying muscle atrophy caused by at least one of the following: aging, disease, and cachexia. The method is also for helping normal muscle contraction, maintaining normal muscle physiology, maintaining normal neuromuscular function, maintaining normal energy metabolism, or enhancing energy level.

Still yet another objective of the present invention is to provide a composition for protecting muscles, wherein the composition is a medicament or a food product comprising an effective amount of the active ingredient or FU-LING extract described above. The composition is for protecting muscle cells against injury, promoting the regeneration and repair of muscles, or for regulating, treating, and/or delaying muscle atrophy caused by at least one of the following: aging, disease, and cachexia. The composition is also for helping normal muscle contraction, maintaining normal muscle physiology, maintaining normal neuromuscular function, maintaining normal energy metabolism, or enhancing energy level.

### BRIEF DESCRIPTION OF THE DRAWINGS

**FIG. 1** illustrates the influence of Tumor necrosis factor alpha (TNF-α) on intracellular reactive oxidative stress (ROS) of C2C12 cells;
**FIG. 2** illustrates the influence of the FU-LING extract on the survival rate of C2C12 cells with TNF-α-induced injury; and
**FIG. 3** illustrates the influence of the FU-LING extract on intracellular reactive oxidative stress of C2C12 cells with TNF-α-induced injury.

### DESCRIPTION OF THE PREFERRED EMBODIMENT

The detailed technology and preferred embodiments implemented for the present invention are described in the following paragraphs for people skilled in the field to well appreciate the features of the claimed invention. However, the present invention may be realized in various embodiments.

In addition, unless otherwise stated herein, the expressions "a," "an," "the" or the like recited in the specification of the present invention (especially in the claims) are intended to include both the singular and plural forms. Furthermore, the term "an effective amount" used in this specification refers to the amount of the compound that can at least partially alleviate the condition in a suspected subject when administered to the subject. The unit "mg/kg-body weight" refers to the dosage in mg required per kg of body weight.

In addition, unless otherwise stated herein, the term "subject" recited in the specification of the present invention (especially in the claims) refers to a mammalian, including human and non-human animals. The terms "treat" and "treating" cover the prevention of particular diseases or disorders, the amelioration of particular diseases or disorders, and/or the prevention or elimination of the diseases or disorders. The term "regulating muscle atrophy" refers to helping normal muscle contraction, maintaining normal muscle physiology, maintaining normal neuromuscular function, maintaining normal energy metabolism and/or enhancing energy level. The term "pharmaceutically acceptable salt" refers to salts that can produce pharmacological activities that are the same as or similar to those produced by their parent compound after being administered to an organism and that are physiologically tolerable (i.e., with a toxicity as low as possible).

The numerical ranges (e.g., 5 to 100) used in this specification should be construed to include all of the rational numbers in the range consisting of any rational numbers in the range. Therefore, the numerical ranges used in this specification should include all the possible combinations of numerical values between the lowest value and the highest value listed therein. In addition, the word "about", "approximately" or "almost" as used herein substantially represents values within ±20% of the stated value, preferably within ±10% and more preferably within ±5%.

Herbal FU-LING refers to the dried sclerotium of *Poria cocos* (Schw.) Wolf, a fungus in the family Fomitopsidaceae. *Poria cocos* fungus often parasitizes on the roots of pine trees. FU-LING is of a light brown or dark brown color at the external layer (the FU-LING epidermis) and of pink or white color at the inner part (the FU-LING meat).

The inventors of the present invention discovered that the FU-LING extract can effectively protect muscle cells against injury, and promote the regeneration and repair of muscles. Thus, the FU-LING extract can be used for protecting muscles and has the effect of regulating, treating and/or delaying muscle atrophy. It is believed that the FU-LING extract used in the present invention can effectively regulate, treat, and/or delay muscle atrophy caused by at least one of the following: aging, disease, and cachexia. Therefore, the present invention provides uses of the FU-LING extract in protecting muscles, comprising using the FU-LING extract in the manufacture of a medicament or a food product for protecting muscles and providing a food product or a pharmaceutical composition comprising the FU-LING extract.

The FU-LING extract adopted in accordance with the present invention can be a liquid extract provided by extracting the FU-LING raw material with a polar solvent. Wherein the FU-LING raw material can be the FU-LING meat, FU-LING epidermis and/or FU-LING fermentation product; the polar solvent can be water and/or a C1-C4 alcohol. Preferably, the polar solvent is water, ethanol, or a combination thereof. The ratio of the amount of the polar solvent and FU-LING can be optionally adjusted. In general, the amount of the polar solvent is not particularly limited as long as the raw material can be uniformly dispersed. In a specific embodiment of the present invention, ethanol is used as the polar solvent, and the raw material is dispersed in ethanol (raw material: ethanol = 1: 8 in volume) to conduct the extraction.

In the above extraction operation, the extraction is conducted for a period of time to achieve the desired degree of extraction. For example, when using ethanol as the polar solvent, the extraction time is usually at least 1 hour, preferably at least 2 hours, more preferably at least 3 hours. The extraction operation can be optionally carried out with other operations such as boiling, cooling, filtration, concentration under vacuum, and resin column chromatography.

Optionally, alkalization treatment and acidification treatment can be conducted sequentially to the liquid extract provided by extracting FU-LING raw material with a polar solvent to enhance the content of tumulosic acid in the liquid extract. The term "alkalization" refers to the use of any suitable alkaline substance to increase the pH value of the material (referring to the liquid extract provided by extracting FU-LING raw material with a polar solvent in the present invention). In a specific embodiment of the present invention, sodium hydroxide is used to conduct the alkalization reaction.

In general, the pH of the material is increased to not less than about 10 in the alkalization treatment step, preferably to about not less than about 11; most preferably to not less than about 12. In a specific embodiment of the present invention, 1N sodium hydroxide is added to increase the pH value of the liquid extract to about 12 in the alkalization treatment. After increasing the pH value of the liquid extract, the alkalization reaction is conducted at an elevated temperature optionally. For example, the alkalization reaction is conducted at a temperature of not less than 50°C, preferably at a temperature of not less than 60°C. As shown in the appended examples, the alkalization reaction can be conducted at a temperature of, for example, about 70°C.

The term "acidification" refers to the use of any suitable acidic substance to reduce the pH value of the material (referring to the above alkalized liquid extract in the present invention). In a specific embodiment of the present invention, hydrochloric acid is used to reduce the pH value of the alkalized liquid extract.

In general, there is no particular requirement for the degree of reduction of pH value in the acidification treatment as long as the pH value is reduced. For example, the pH value of the liquid extract is reduced by at least about 3.0, preferably at least about 4.0. In a specific embodiment of the present invention, the pH value of the alkalized liquid extract is reduced to about 7 in the acidification treatment.

The FU-LING extract adopted in accordance with the present invention can also be a dry substance which can be provided by drying the aforementioned liquid extract obtained by the alkalization and acidification treatment or without alkalization and acidification treatment. To achieve maximum extraction efficiency, FU-LING raw material can optionally be extracted repeatedly with the same or different polar solvents, and the liquid extracts thus obtained can be combined, and then dried, or optionally be alkalized or acidified. Each extraction process can be conducted by using the same or different polar solvents.

The inventors of the present invention further found that, among all of the ingredients of the FU-LING extract, tumulosic acid itself can effectively promote the regeneration and repair of muscles, thus can be used for protecting muscles and has the effect of regulating, treating and/or delaying muscle atrophy. As shown in the appended examples, it is believed that tumulosic acid can effectively regulate, treat, and/or delay muscle atrophy caused by at least one of the following: aging, disease, and cachexia. Therefore, the present invention also provides uses of tumulosic acid and/or a pharmaceutically acceptable salt of tumulosic acid in protecting muscles, including using tumulosic acid and/or a pharmaceutically acceptable salt of tumulosic acid in the manufacture of a medicament or a food product for protecting muscles and providing a food product or a pharmaceutical composition comprising tumulosic acid and/or a pharmaceutically acceptable salt of tumulosic acid.

Preferably, the tumulosic acid and/or a pharmaceutically acceptable salt of tumulosic acid are used as a plant extract; and more preferably, used as a FU-LING extract, especially used as a polar solvent extract of FU-LING, wherein the polar solvent is selected from the group consisting of water, C1-C4 alcohols, and combinations thereof.

Depending on the desired purpose(s), the pharmaceutical composition or the medicament provided in accordance with the present invention could be provided in any suitable form For example, the pharmaceutical composition or the medicament can be administered by an oral or parenteral (such as subcutaneous, intravenous, intramuscular, peritoneal, or nasal) route to a subject in need to treat, and/or delay muscle atrophy caused by aging, disease and/or cachexia. Depending on the form and purpose(s), a suitable carrier could be chosen and used to provide the pharmaceutical composition or the medicament. Examples of the carrier include excipients, diluents, auxiliaries, stabilizers, absorbent retarders, disintegrating agent, hydrotropic agents, emulsifiers, antioxidants, adhesives, binders, tackifiers, dispersants, suspending agents, lubricants, hygroscopic agents, etc.

As a form suitable for oral administration, the pharmaceutical composition or the medicament provided in accordance with the present invention could comprise any pharmaceutically acceptable carrier that will not adversely affect the desired effects of the active ingredient (i.e., the FU-LING extract or tumulosic acid). For example, the pharmaceutically acceptable carrier can be water, saline, dextrose, glycerol, ethanol or its analogs, cellulose, starch, sugar bentonite, and combinations thereof. The pharmaceutical composition or the medicament can be provided by any suitable method in any suitable form for oral administration, such as in the form of a tablet (e.g., sugar-coated tablet), a pill, a capsule, granules, a pulvis, a fluidextract, a solution, syrup, a suspension, a tincture, etc.

As for the form of injections or drips suitable for subcutaneous, intravenous, intramuscular, or peritoneal administration, the pharmaceutical composition or the medicament provided in accordance with the present invention could comprise one or more ingredient(s), such as an isotonic solution, a salt-buffered saline (e.g., phosphate-buffered saline or citrate-buffered saline), a hydrotropic agent, an emulsifier, 5% sugar solution, and other carriers to provide the pharmaceutical composition or the medicament as an intravenous infusion, an emulsified intravenous infusion, a powder for injection, a suspension for injection, or a powder suspension for injection, etc. Alternatively, the pharmaceutical composition or the medicament could be prepared as a pre-injection solid. The pre-injection solid can be provided in a form which is soluble in other solutions or suspensions, or in an emulsifiable form. A desired injection is provided by dissolving the pre-injection solid in other solutions or suspensions or emulsifying it prior to being administered to a subject in need.

Optionally, the pharmaceutical composition or the medicament provided in accordance with the present invention could further comprise a suitable amount of additives, such as a flavoring agent, a toner, or a coloring agent for enhancing the palatability and the visual perception of the pharmaceutical composition or the medicament, and/or a buffer, a conservative, a preservative, an antibacterial agent, or an antifungal agent for improving the stability and storability of the pharmaceutical composition or the medicament. In addition, the pharmaceutical composition or the medicament could optionally further comprise one or more other active ingredient(s) (such as vitamin D, vitamin B1, vitamin B2, nicotine, biotin, pantothenic acid, calcium, iodine, magnesium, zinc, proteins, etc.), or be used in combination with a medicament comprising one or more other active ingredients, to further enhance the effects of the pharmaceutical composition or the medicament, or to increase the application flexibility and adaptability of the preparation thus provided, as long as the other active ingredients do not adversely affect the desired effects of the active ingredient of the present invention (i.e., the FU-LING extract or tumulosic acid).

Depending on the needs, age, body weight, and health conditions of the subject, the pharmaceutical composition or the medicament provided in accordance with the present invention could be dosed at various administration frequencies, such as once a day, multiple times a day, or once every few days, etc. For example, when the pharmaceutical composition or the medicament is administered orally to a subject for protecting muscles, the dosage of the pharmaceutical composition or the medicament, as a FU-LING extract, is about 0.003 mg/kg-body weight to about 30 mg/kg-body weight per day, preferably about 0.05 mg/kg-body weight to about 20 mg/kg-body weight per day, and more preferably about 1 mg/kg-body weight to about 15 mg/kg-body weight per day. Alternatively, the dosage of the pharmaceutical composition or the medicament, as tumulosic acid, is about 0.5 mg/kg-body weight to about 20 mg/kg-body weight per day, preferably about 1 mg/kg-body weight to about 15 mg/kg-body weight per day, and more preferably about 5 mg/kg-body weight to about 10 mg/kg-body weight per day.

The food product provided in accordance with the present invention is useful for helping normal muscle contraction, maintaining normal muscle physiology, maintaining normal neuromuscular function, maintaining normal energy metabolism, or enhancing energy level, and can be used for regulating muscle atrophy caused by aging, disease and/or cachexia.

The food product provided in accordance with the present invention could be a health food, a nutritional supplement food or a special nutrition food, and it could be provided as dairy products, processed meat products, breadstuff, pasta, cookies, troche, capsules, fruit juices, teas, sport drinks, nutritional drinks, etc., Preferably, the food product of the present invention is a health food.

Depending on the age, body weight, amount of exercise and health conditions of the subject, the recommended daily dosage of the health food, nutritional supplement food and special nutrition food provided in accordance with the present invention can be taken at various frequencies, such as once a day, several times a day or once every few days, etc. Also, the amount of the FU-LING extract or tumulosic acid in the health food, nutritional supplement food and special nutrition food provided in accordance with the present invention could be adjusted, preferably to the amount that should be taken daily, depending on the specific population.

The recommended daily dosage, use standards and use conditions for a specific population (e.g., person with considerable exercises, pregnant women, cancer patients, and heart failure patients), or the recommendations for a use in combination with other food product or medicament can be indicated on the exterior package of the health food, nutritional supplement food and/or special nutrition food of the present invention. Thus, it is suitable for the user to take the health food, nutritional supplement food and/or special nutrition food by him- or herself safely and securely without the instructions of a doctor, a pharmacist, or related advisors.

Described is a method for protecting muscles, comprising administering to a subject in need of an effective amount of an active ingredient, wherein the active ingredient is a FU-LING extract, tumulosic acid and/or a pharmaceutically acceptable salt of tumulosic acid. In the method for protecting muscles in accordance with the present invention, the applied route, applied form, suitable dosage and uses of the active ingredients are all in line with the above descriptions.

The present invention will be further illustrated in detail with specific examples as follows. The scope of the present invention will be indicated in the appended claims.

### Example 1: Preparation and ingredient analysis of the FU-LING extract

**(1-1)**
Herbal FU-LING (habitat: Yunnan, China) was washed, and its skin was peeled (hereinafter referred to as "FU-LING epidermis"), and the rest was meat part (hereinafter referred to as "FU-LING meat").
**(1-2)**
700 kg of the FU-LING meat obtained from (1-1) was soaked in 75% ethanol (FU-LING: 75% ethanol = 1: 8 in volume) at room temperature for 12 hours, and then stewed for 3 hours to provide a liquid extract. The foregoing extraction procedures were repeated three times. The liquid extracts obtained from the three extractions were combined and filtered to remove insoluble and provide a filtrate. The ethanol contained in the filtrate was removed by concentration under vacuum to provide a concentrated solution. The concentrated solution was spray-dried by a spray dryer to provide a FU-LING extract.
**(1-3)**
The components of the extract obtained from (1-2) were detected by LC/UV/MS (liquid chromatography coupled to diode array UV detection and mass spectrometry) at the wavelengths of 243 nm and 210 nm, respectively. The results showed that the extract obtained from (1-2) comprised pachymic acid, dehydropachymic acid, tumulosic acid, dehydrotumulosic acid, polyporenic acid C and 3-epi-dehydrotumulosic acid. Then, the content of each component in the extract was quantified by high performance liquid chromatography (HPLC), and the results are shown in Table 1.

**Table 1**

| | Pachymic acid | Dehydro- pachymic acid | Tumulosic acid | Dehydro- tumulosic acid | Poly- porenic acid C | 3-epi-dehydro tumulosic acid |
|---|---|---|---|---|---|---|
| Content in the extract (%) | 7.07 | 2.54 | 7.47 | 3.76 | 1.63 | 1.02 |

**(1-4)**
3250g of the FU-LING extract obtained from (1-2) was uniformly dispersed in pure water (FU-LING: pure water = 1: 10 in volume). Then, sodium hydroxide was added to provide the mixture with an alkali concentration of 1N (i.e., the pH value of the mixture was increased to about 12). The mixture was poured into a mixing barrel which maintained at 70 °C and was stirred uniformly until the reaction was complete. Then, the mixture was neutralized with 12N concentrated hydrochloric acid to decrease the pH value of the mixture to 7 and centrifuged at 1000 rpm (by flatbed centrifuge) at room temperature for 30 minutes to remove the filtrate, and the remaining insoluble was washed with pure water. The insoluble was dried and ground to provide a powder. The powder was extracted with 95% ethanol (insoluble powder: 95% ethanol = 1: 40 in volume), and the foregoing extraction procedures were repeated three times. The liquid extracts obtained from the three extractions were combined and then concentrated under vacuum to remove ethanol to obtain 2000g of the product (namely, FU-LING meat extract used in the following experiments).
**(1-5)**
The components of the extract obtained from (1-4) were detected by LC/UV/MS at the wavelengths of 243 nm and 210 nm, respectively. The results showed that the extract obtained from (1-4) comprised tumulosic acid, dehydrotumulosic acid, polyporenic acid C and 3-epi-dehydrotumulosic acid, but did not comprise pachymic acid and dehydropachymic acid. Then, the content of each component in the extract was quantified by high performance liquid chromatography. The results showed that the total content of tumulosic acid, dehydrotumulosic acid, polyporenic acid C and 3-epi-dehydrotumulosic acid in the extract obtained from (1-4) was 6.2 wt%. Wherein, based on the total weight of tumulosic acid, dehydrotumulosic acid, polyporenic acid C and 3-epi-dehydrotumulosic acid, tumulosic acid accounted for about 53.74 wt%, dehydrotumulosic acid accounted for about 21.26 wt%, polyporenic acid C accounted for about 12.10 wt% and 3-epi-dehydrotumulosic acid accounted for about 12.90 wt% respectively.
**(1-6)**
The FU-LING epidermis obtained from (1-1) was soaked in 75% ethanol (FU-LING epidermis: 75% ethanol = 1: 8 in volume) at room temperature for 12 hours, and then stewed for 3 hours to provide a liquid extract. The foregoing extraction procedures were repeated three times. The liquid extracts obtained from the three extractions were combined and filtered to remove the insoluble and provide a filtrate. The ethanol contained in the filtrate was removed by concentration under vacuum to provide a concentrated solution. The concentrated solution was spray-dried by a spray dryer to provide a FU-LING epidermis extract for the following experiments.
**(1-7)**
The FU-LING strain was inoculated in a sterilized solid matrix and fermented in the dark environment at a temperature of 20 to 35°C and a relative humidity of 30 to 70% for several weeks to several months to obtain a FU-LING fermentation product. 100g of the above FU-LING fermentation product was dried and ground to provide a powder. Then, the powder was uniformly dispersed in 75% ethanol (powder of the fermentation product: 75% ethanol = 1: 8 in volume) at room temperature for 12 hours, and then stewed for 3 hours to provide a liquid extract, and the foregoing extraction procedures were repeated three times. The liquid extracts obtained from the three extractions were combined and filtered to remove insoluble and provide a filtrate. The ethanol contained in the filtrate was removed by concentration under vacuum to provide a concentrated solution. The concentrated solution was spray-dried by a spray dryer to provide a FU-LING fermentation extract for the following experiments.

### Example 2: Establishment of a muscle cell injury model

Tumor necrosis factor alpha (TNF-α) is a pro-inflammatory cytokine with a molecular weight of 17,000. Human clinical data have shown that the level of TNF-α increases in patients with special diseases (e.g., cancer, AIDS or COPD), patients using anti-cancer drugs, and the elderly, which is also associated with an increment in muscle catabolism (i.e., the decomposition and consumption of muscle) or muscle cell apoptosis. It was revealed by researchers that an injection of TNF-α or an administration of the drug that would increase the TNF-α level in an animal body may induce muscle cell injury (including the metabolic imbalance of muscle protein, muscle cell apoptosis, etc.), and further cause muscle atrophy. To investigate the effects and mechanisms of the FU-LING extract and the ingredients therein on protecting muscles, the inventors of the present invention established a muscle cell injury model by using TNF-α.

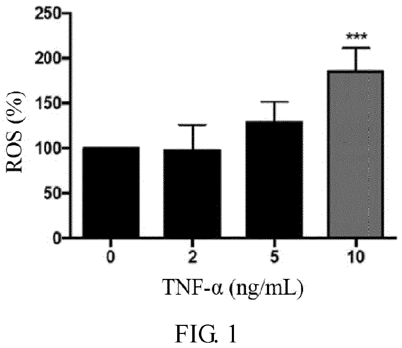

Firstly, C2C12 cells (i.e., myoblasts of mice, purchased from ATCC) were cultivated in H-DMEM medium (purchased from Sigma company) until 80% confluence was attained (i.e., the mixed cell monolayer comprised 80% of the area). Thereafter, the cells were separated into four groups, and the medium of each group was replaced with a differentiation medium supplemented with 2% horse serum. TNF-α (purchased from Sigma company) was then added into the medium to provide final concentrations of 0, 2, 5 or 10 ng/mL respectively. After being co-treated with the differentiation medium and TNF-α for 4 days, the intracellular reactive oxidative stress (ROS) of the C2C12 cells was measured to serve as the index for model evaluation. Accordingly, a TNF-α-induced muscle cell injury model was established. Finally, the result of the group that was not treated with TNF-α (i.e., the concentration of TNF-α was 0 ng/mL) served as a basis to calculate the relative intracellular ROS of the other groups. The results are shown in Figure 1 (all the data are presented as average values ± SEM, n = 6, analyzed with a t-test, *** p<0.001).

As shown in Figure 1, the ROS in the C2C12 cells treated with 5 ng/mL of TNF-α increased; and the ROS in the C2C12 cells treated with 10 ng/mL of TNF-α increased more significantly. Therefore, 10 ng/mL was chosen as the experimental concentration of TNF-α for inducing muscle cell injury in the following experiments.

### Example 3: Effects of the FU-LING extract on protecting muscle cells against injury

The FU-LING extract obtained from Example 1 (1-4) was formulated as a FU-LING meat extract solution in dimethyl sulfoxide (DMSO; purchased from Sigma). After 80% confluence of cells was attained in H-DMEM medium, cells were separated into five groups and treated as follows:
(1) Control group: Cells were cultivated in H-DMEM medium (free of FU-LING extract) for 6 hours; thereafter, the medium was replaced with a differentiation medium supplemented with 2% horse serum, and the cells were cultivated for 4 days.
(2) TNF-α group: Cells were cultivated in H-DMEM medium for 6 hours; thereafter, the medium was replaced with a differentiation medium supplemented with 2% horse serum, and TNF-α was then added into the medium (the final concentration of TNF-α in the medium was 10 ng/mL) to conduct a co-treatment with the differentiation medium for 4 days.
(3) TNF-α+ FU-LING extract group (three groups in total): The FU-LING extract solution was added to H-DMEM mediums to provide final concentrations of 1, 5 or 10 µg/mL, respectively. The cells were pre-treated with the above mediums for 6 hours. Thereafter, the mediums were replaced with differentiation mediums supplemented with 2% horse serum, and TNF-α was then added to the mediums (the final concentration of TNF-α in the medium was 10 ng/mL) to conduct co-treatments with differentiation mediums for 4 days.

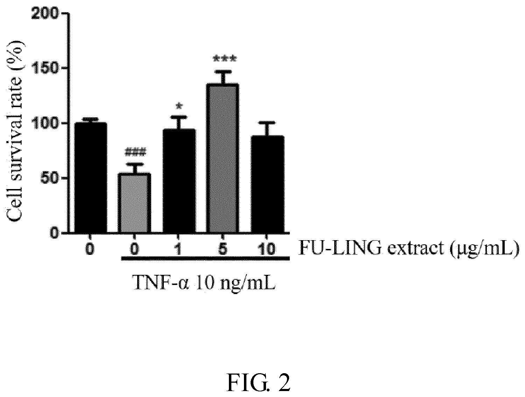

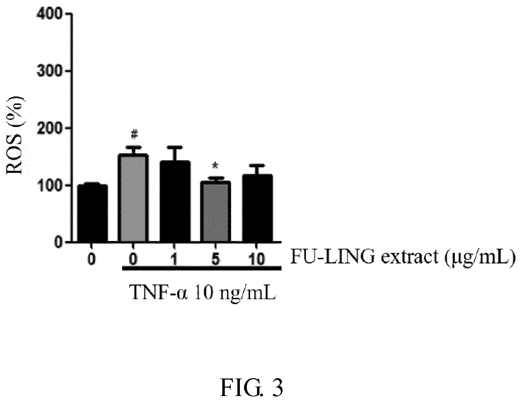

The survival rate (tested by MTT assay) and intracellular ROS of the C2C12 cells in each group were measured. The result of control group was served as a basis to calculate the relative survival rates and intracellular ROS of the other groups to evaluate the effective concentration of the FU-LING extract on protecting the TNF-α-induced muscle cell injury. The results are shown in Figures 2 and 3 (all the data are presented as average values ± SEM, n = 6, analyzed with a t-test, # p<0.05, ### p<0.001, * p<0.05, *** p<0.001).

As shown in Figures 2 and 3, in the group with TNF-α-induced cell injury, when cells were pre-treated with FU-LING extract ranging from 1 to 5 µg/mL, the survival rates of C2C12 cells gradually increased along with the increment in the concentration of FU-LING extract, and intracellular ROS of C2C12 cells gradually decreased along with the increment in the concentration of FU-LING extract. When cells were pre-treated with 5 µg/mL of FU-LING extract, the ROS decreased to be equivalent to that in the cells of the control group (without TNF-α-induced injury). The above results indicate that the FU-LING extract can effectively decrease the muscle cell injury caused by TNF-α, and has the effect of protecting muscle cells against injury.

### Example 4: Effects of the FU-LING extract and tumulosic acid on promoting the regeneration and repair of muscles

As described above, myogenin and MyHC can be used as markers for myoblast differentiation. To investigate whether FU-LING extract and tumulosic acid have the effect on promoting differentiation of myoblasts, tumulosic acid (purchased from Sinphar pharmaceutical co. ltd), FU-LING meat extract obtained from Example 1 (1-4), FU-LING epidermis extract obtained from Example 1 (1-6), and the FU-LING fermentation extract obtained from Example 1 (1-7) were formulated into the following: (1) tumulosic acid solution, (2) FU-LING meat extract solution, (3) FU-LING epidermis extract solution, and (4) FU-LING fermentation extract solution, respectively, in dimethyl sulfoxide (DMSO; purchased from Sigma Company).

After 80% confluence of cells was attained in H-DMEM medium, cells were separated into fourteen groups and treated as follows:
(1) Control group: The medium was replaced with a differentiation medium supplemented with 2% horse serum, and the cells were cultivated for 4 days.
(2) Meat extract group (three groups in total): The medium was replaced with a differentiation medium supplemented with 2% horse serum, and FU-LING meat extract solution was then added to the medium to a final concentration of 0.1, 1 or 10 µg/mL to conduct a treatment for 4 days.
(3) Epidermis extract group (four groups in total): The medium was replaced with a differentiation medium supplemented with 2% horse serum, and FU-LING epidermis extract solution was then added to the medium to a final concentration of 0.01, 0.1, 0.5 or 1 µg/mL to conduct a treatment for 4 days.
(4) Fermentation extract group (three groups in total): The medium was replaced with a differentiation medium supplemented with 2% horse serum, and FU-LING fermentation extract solution was then added to the medium to a final concentration of 0.2, 2, or 20 µg/mL to conduct a treatment for 4 days.
(5) Tumulosic acid group (four groups in total): The medium was replaced with a differentiation medium supplemented with 2% horse serum, and tumulosic acid solution was then added to the medium to a final concentration of 0.01, 0.1, 1 or 10 µg/mL) to conduct a treatment for 4 days.

Thereafter, proteins of the cells in each group were extracted, and the expression levels of myoblast differentiation markers (myogenin and MyHC) were detected by Western blot. Finally, the result of control group served as a basis (i.e., the expression level of the control group was set as 100%) to calculate the relative expression levels of myogenin and MyHC proteins of the cells in each group. The results are shown in Table 2.

**Table 2**

| Groups | | Expression level of myogenin (%) | Expression level of MyHC (%) |
|---|---|---|---|
| Control group | | 100 | 100 |
| Meat extract group (µg/mL) | 0.1 | 130 | 110 |
| | 1 | 100 | 120 |
| | 10 | 270 | 130 |
| Epidermis extract group (µg/mL) | 0.01 | 150 | 110 |
| | 0.1 | 180 | 140 |
| | 0.5 | 170 | 210 |
| | 1 | 140 | 220 |
| Fermentation extract group (µg/mL) | 0.2 | 170 | 160 |
| | 2 | 220 | 130 |
| | 20 | 160 | 190 |
| Tumulosic acid group (µg/mL) | 0.01 | 130 | 90 |
| | 0.1 | 130 | 80 |
| | 1 | 160 | 70 |
| | 10 | 490 | 180 |

As shown in Table 2, a low-dose (0.1 µg/mL) of FU-LING meat extract can increase the expression level of myogenin protein by about 30%, and a high-dose (10 µg/mL) of FU-LING meat extract can increase the expression level of myogenin protein by up to 170%. In another aspect, a low-dose (0.01 µg/mL) of the FU-LING epidermis extract can increase the expression level of myogenin protein by about 50%, 0.1 to 0.5 µg/mL of FU-LING epidermis extract can increase the expression level of myogenin protein by about 70 to 80% and 1 µg/mL of FU-LING epidermis extract also increased the expression level of myogenin protein. In still another aspect, a low-dose (0.2 µg/mL) of FU-LING fermentation extract can increase the expression level of myogenin protein by about 70%, 2 µg/mL of FU-LING fermentation extract can increase the expression level of myogenin protein by up to 120%, and 20 µg/mL of FU-LING fermentation extract also increased the expression level of myogenin protein. In still another aspect, a low-dose (0.01 to 0.1 µg/mL) of tumulosic acid can increase the expression level of myogenin protein by about 30%. The expression level of myogenin protein increased along with the increment in the concentration of tumulosic acid, wherein 10 µg/mL of tumulosic acid increased the expression level of myogenin protein by about 390%.

Also as shown in Table 2, 10 µg/mL of FU-LING meat extract can increase the expression level of MyHC protein by about 30%; in another aspect, a low-dose (0.01 µg/mL) of the FU-LING epidermis extract can increase the expression level of MyHC protein by 10%, and the expression level of MyHC protein increased along with the increment in the concentration of FU-LING epidermis extract, wherein 1 µg/mL of FU-LING epidermis extract can increase the expression level of MyHC protein by about 120%. In still another aspect, a low-dose (0.2 µg/mL) of FU-LING fermentation extract can increase the expression level of MyHC protein by about 60%, and 20 µg/mL of FU-LING fermentation extract can increase the expression level of MyHC protein by up to 90%. In still another aspect, 10 µg/mL of tumulosic acid can increase the expression level of MyHC protein by about 80%.

The above results showed that FU-LING extract (including FU-LING meat extract, FU-LING epidermis extract, FU-LING fermentation extract) and tumulosic acid can both effectively increase the expression levels of myoblast differentiation markers (myogenin and MyHC) in C2C12 cells, therefore, can provide the effect of promoting muscle cell differentiation, and are useful for helping the regeneration and repair of the injured muscle tissues.
As shown in the above experimental results, the FU-LING extract can effectively protect the injured muscle cells, thereby increasing the muscle cell survival rate and simultaneously inhibiting the increment in ROS. Therefore, the FU-LING extract has the effect on ameliorating the oxidative stress of injured muscle cells. Furthermore, the FU-LING extract and tumulosic acid contained therein can promote the differentiation of myoblasts. Given the above, the FU-LING extract and ingredients contained therein can effectively protect muscle cells against injury, promote the regeneration and repair of muscles. Hence, the FU-LING extract and ingredients contained therein can be used for protecting muscles, and are useful for helping normal muscle contraction, maintaining normal muscle physiology, maintaining normal neuromuscular function, maintaining normal energy metabolism, or enhancing energy level.

## Claims

1. A FU-LING (Poria cocos) extract for use in treating, delaying and/or regulating muscle atrophy by protecting muscle cells against injury and/or promoting the regeneration and repair of muscles.

2. The FU-LING extract for use as claimed in Claim 1, wherein the FU-LING extract is a polar solvent extract of FU-LING, and the polar solvent is selected from the group consisting of water, C1-C4 alcohols, and combinations thereof.

3. The FU-LING extract for use as claimed in Claim 1 or 2, wherein the FU-LING extract comprises tumulosic acid.

4. The FU-LING extract for use as claimed in any one of Claims 1 to 3, wherein the FU-LING extract is an extract of FU-LING meat, an extract of FU-LING epidermis and/or an extract of FU-LING fermentation product.

5. The FU-LING extract for use as claimed in any one of Claims 1 to 4, wherein the FU-LING extract is for treating, and/or delaying muscle atrophy caused by at least one of the following: aging, disease, and cachexia.

6. The FU-LING extract for use as claimed in any one of Claims 1 to 4, wherein the FU-LING extract is for regulating muscle atrophy caused by at least one of the following: aging, disease, and cachexia.

7. Non-therapeutic use of a FU-LING (Poria cocos) extract in a food product for helping normal muscle contraction, maintaining normal muscle physiology, maintaining normal neuromuscular function, maintaining normal energy metabolism, or enhancing energy level, wherein the food product is a health food, a nutrient supplementary food, or a special nutrition food.

8. An active ingredient for use in treating, delaying and/or regulating muscle atrophy by protecting muscle cells against injury and/or promoting the regeneration and repair of muscles, wherein the active ingredient is tumulosic acid and/or a pharmaceutically acceptable salt of tumulosic acid.

9. The active ingredient for use as claimed in Claim 8, wherein the active ingredient is used as a plant extract.

10. The active ingredient for use as claimed in Claim 8 or 9, wherein the active ingredient is for treating, and/or delaying muscle atrophy caused by at least one of the following: aging, disease, and cachexia.

11. The active ingredient for use as claimed in Claim 8 or 9, wherein the active ingredient is for regulating muscle atrophy caused by at least one of the following: aging, disease, and cachexia.

12. Non-therapeutic use of an active ingredient in a food product for helping normal muscle contraction, maintaining normal muscle physiology, maintaining normal neuromuscular function, maintaining normal energy metabolism, or enhancing energy level, wherein the active ingredient is tumulosic acid and/or a pharmaceutically acceptable salt of tumulosic acid, and wherein the food product is a health food, a nutrient supplementary food, or a special nutrition food.

## Patentansprüche

1. Extrakt des Kiefernschwamms (Fu Ling, *Porio cocos*) zur Verwendung bei der Behandlung, Verzögerung und/oder Regulierung von Muskelschwund, indem die Muskelzellen vor Verletzungen geschützt werden und/oder die Regeneration und Reparatur von Muskeln gefördert werden.

2. Extrakt des Kiefernschwamms zur Verwendung gemäß Anspruch 1, wobei der Extrakt des Kiefernschwamms ein Kiefernschwammextrakt in einem polaren Lösungsmittel ist und das polare Lösungsmittel aus der Gruppe ausgewählt ist, die aus Wasser, C₁-C₄-Alkoholen und Kombinationen davon besteht.

3. Extrakt des Kiefernschwamms zur Verwendung gemäß Anspruch 1 oder 2, wobei der Extrakt des Kiefernschwamms Polyporensäure umfasst.

4. Extrakt des Kiefernschwamms zur Verwendung gemäß einem der Ansprüche 1 bis 3, wobei der Extrakt des Kiefernschwamms ein Extrakt von Kiefernschwammfleisch, ein Extrakt von Kiefernschwamm-Epidermis und/oder ein Extrakt von Kiefernschwamm-Fermentationsprodukt ist.

5. Extrakt des Kiefernschwamms zur Verwendung gemäß einem der Ansprüche 1 bis 4, wobei der Extrakt des Kiefernschwamms zur Behandlung und/oder Verzögerung von Muskelschwund bestimmt ist, der wenigstens eine der folgenden Ursachen hat: Alter, Krankheit und Kachexie.

6. Extrakt des Kiefernschwamms zur Verwendung gemäß einem der Ansprüche 1 bis 4, wobei der Extrakt des Kiefernschwamms zur Regulierung von Muskelschwund bestimmt ist, der wenigstens eine der folgenden Ursachen hat: Alter, Krankheit und Kachexie.

7. Nichttherapeutische Verwendung eines Extrakts des Kiefernschwamms (Fu Ling, *Porio cocos*) in einem Lebensmittel zur Unterstützung der normalen Muskelkontraktion, zur Aufrechterhaltung der normalen Muskelphysiologie, zur Aufrechterhaltung der normalen neuromuskulären Funktion, zur Aufrechterhaltung des normalen Energiestoffwechsels oder zur Erhöhung des Energieniveaus, wobei das Lebensmittel ein Health Food, ein Nahrungsergänzungsmittel oder ein Lebensmittel für spezielle Ernährung ist.

8. Wirkstoff zur Verwendung bei der Behandlung, Verzögerung und/oder Regulierung von Muskelschwund, indem die Muskelzellen vor Verletzungen geschützt werden und/oder die Regeneration und Reparatur von Muskeln gefördert werden, wobei es sich bei dem Wirkstoff um Polyporensäure und/oder ein pharmazeutisch annehmbares Salz von Polyporensäure handelt.

9. Wirkstoff zur Verwendung gemäß Anspruch 8, wobei der Wirkstoff als Pflanzenextrakt verwendet wird.

10. Wirkstoff zur Verwendung gemäß Anspruch 8 oder 9, wobei der Wirkstoff zur Behandlung und/oder Verzögerung von Muskelschwund bestimmt ist, der wenigstens eine der folgenden Ursachen hat: Alter, Krankheit und Kachexie.

11. Wirkstoff zur Verwendung gemäß Anspruch 8 oder 9, wobei der Wirkstoff zur Regulierung von Muskelschwund bestimmt ist, der wenigstens eine der folgenden Ursachen hat: Alter, Krankheit und Kachexie.

12. Nichttherapeutische Verwendung eines Wirkstoffs in einem Lebensmittel zur Unterstützung der normalen Muskelkontraktion, zur Aufrechterhaltung der normalen Muskelphysiologie, zur Aufrechterhaltung der normalen neuromuskulären Funktion, zur Aufrechterhaltung des normalen Energiestoffwechsels oder zur Erhöhung des Energieniveaus, wobei es sich bei dem Wirkstoff um Polyporensäure und/oder ein pharmazeutisch annehmbares Salz von Polyporensäure handelt und wobei das Lebensmittel ein Health Food, ein Nahrungsergänzungsmittel oder ein Lebensmittel für spezielle Ernährung ist.

## Revendications

1. Extrait de pachyme (Fu ling, *Poria cocos*) à utiliser pour traiter, retarder et/ou réguler l'atrophie musculaire en protégeant les cellules musculaires contre les blessures et/ou en favorisant la régénération et la réparation des muscles.

2. Extrait de pachyme à utiliser comme revendiqué dans la revendication 1, dans lequel ledit extrait de pachyme est un extrait de solvant polaire de pachyme, et ledit solvant polaire est choisi dans le groupe consistant en eau, alcools en C₁-C₄, et des combinaisons de ceux-ci.

3. Extrait de pachyme à utiliser comme revendiqué dans la revendication 1 ou 2, dans lequel ledit extrait de pachyme comprend l'acide tumulosique.

4. Extrait de pachyme à utiliser comme revendiqué dans l'une quelconque des revendications 1 à 3, dans lequel ledit extrait de pachyme est un extrait de chair de pachyme, un extrait de l'épiderme de pachyme et/ou un extrait de produit de fermentation de pachyme.

5. Extrait de pachyme à utiliser comme revendiqué dans l'une quelconque des revendications 1 à 4, dans lequel ledit extrait de pachyme est destiné à traiter et/ou à retarder l'atrophie musculaire causée par au moins l'un des éléments suivants : vieillissement, maladie et cachexie.

6. Extrait de pachyme à utiliser comme revendiqué dans l'une quelconque des revendications 1 à 4, dans lequel ledit extrait de pachyme est destiné à réguler l'atrophie musculaire causée par au moins l'un des éléments suivants : vieillissement, maladie et cachexie.

7. Utilisation non thérapeutique d'un extrait de pachyme (*Poria cocos*) dans un produit alimentaire pour favoriser la contraction musculaire normale, maintenir une physiologie musculaire normale, maintenir une fonction neuromusculaire normale, maintenir un métabolisme énergétique normal ou améliorer le niveau d'énergie, dans laquelle le produit alimentaire est un aliment santé, un aliment complémentaire nutritif ou un aliment nutritionnel spécial.

8. Principe actif à utiliser pour traiter, retarder et/ou réguler l'atrophie musculaire en protégeant les cellules musculaires contre les blessures et/ou en favorisant la régénération et la réparation des muscles, dans lequel ledit principe actif est un acide tumulosique et/ou un sel pharmaceutiquement acceptable de l'acide tumulosique.

9. Principe actif à utiliser comme revendiqué dans la revendication 8, dans lequel ledit principe actif est utilisé en tant qu'extrait de plante.

10. Principe actif à utiliser comme revendiqué dans la revendication 8 ou 9, dans lequel ledit principe actif est destiné à traiter et/ou à retarder l'atrophie musculaire causée par au moins l'un des éléments suivants : vieillissement, maladie et cachexie.

11. Principe actif à utiliser comme revendiqué dans la revendication 8 ou 9, dans lequel ledit principe actif est destiné à réguler l'atrophie musculaire causée par au moins l'un des éléments suivants : vieillissement, maladie et cachexie.

12. Utilisation non thérapeutique d'un principe actif dans un produit alimentaire pour favoriser la contraction musculaire normale, maintenir une physiologie musculaire normale, maintenir une fonction neuromusculaire normale, maintenir un métabolisme énergétique normal ou améliorer le niveau d'énergie, dans lequel ledit principe actif est l'acide tumulosique et/ou un sel pharmaceutiquement acceptable de l'acide tumulosique, et dans laquelle le produit alimentaire est un aliment santé, un aliment complémentaire nutritif ou un aliment nutritionnel spécial.
